(19) Europäisches Patentamt
European Patent Office
Office européen des brevets

(11) **EP 3 763 226 A1**

(12) **EUROPÄISCHE PATENTANMELDUNG**

(43) Veröffentlichungstag:
**13.01.2021 Patentblatt 2021/02**

(21) Anmeldenummer: **19185940.4**

(22) Anmeldetag: **12.07.2019**

(51) Int Cl.:
*A23L 33/105* (2016.01)  *A23L 33/115* (2016.01)
*A61K 45/00* (2006.01)  *A61P 17/14* (2006.01)
*A61Q 7/00* (2006.01)  *A61K 36/42* (2006.01)
*A61K 36/55* (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Merz Pharma GmbH & Co. KGaA
60318 Frankfurt am Main (DE)**

(72) Erfinder:
• **Hülsmann, Olaf
  42659 Solingen (DE)**
• **Berlekamp, Uwe
  48432 Rheine (DE)**
• **Becker, Kirstin
  60316 Frankfurt am Main (DE)**

(74) Vertreter: **Banemann, Andreas et al
Merz Pharma GmbH & Co. KGaA
Eckenheimer Landstraße 100
60318 Frankfurt am Main (DE)**

(54) **ZUSAMMENSETZUNG ZUR VERMINDERUNG VON HAARAUSFALL UND ZUR VERBESSERUNG DES HAARWUCHSES BEI ANDROGENETISCHER ALOPEZIE**

(57)    Die vorliegende Erfindung betrifft eine oral einzunehmende Zusammensetzung, insbesondere geeignet als Nahrungsergänzungsmittel oder Diätetisches Lebensmittel, wobei die Zusammensetzung Kakao Extrakt, Leinsamen Extrakt und Kürbiskernöl umfasst und dessen Verwendung zur Verminderung vom Haarausfall und zur Verbesserung des Haarwuchses bei androgenetischer Alopezie.

EP 3 763 226 A1

**Beschreibung**

**Technisches Gebiet**

Die vorliegende Erfindung betrifft eine oral einzunehmende Zusammensetzung, insbesondere geeignet als Nahrungsergänzungsmittel oder Diätetisches Lebensmittel, wobei die Zusammensetzung Kakao Extrakt, Leinsamen Extrakt und Kürbiskernöl umfasst und die Verwendung einer Kombination aus Kakao Extrakt, Leinsamen Extrakt und Kürbiskernöl zur Verminderung vom Haarausfall und zur Verbesserung des Haarwuchses bei androgenetischer Alopezie.

**Beschreibung**

Die androgenetische Alopezie (Alopecia androgenetica, AGA) ist eine erblich bedingte Form des Haarausfalls, die sowohl Männer als auch Frauen betreffen kann. Sie ist gekennzeichnet durch eine erhöhte Empfindlichkeit des Haarfollikels gegenüber männlichen Geschlechtshormonen (Androgenen), vor allem Dihydrotestosteron (DHT), das in den Follikeln durch $5\alpha$-Reduktase aus Testosteron gebildet wird. Mit einem ungefähren Anteil von bis zu 80% bei Männern und 42% bei Frauen ist die androgenetische Alopezie der häufigste Grund für Haarausfall (Blumeyer et al. J Dtsch Dermatol Ges 2011;9 Suppl 6:S1-57). Die Häufigkeit der AGA nimmt mit steigendem Lebensalter zu, wobei sie jedoch vor allem bei Männern auch bereits mit der Pubertät auftreten kann. Der Haarausfall betrifft nur den Kopf, folgt einem typischen Manifestationsmuster, das sich bei Männern und Frauen unterscheidet, und führt zu einer chronisch fortschreitenden Verringerung der Haardichte. Obwohl die androgenetische Alopezie nicht gesundheitsgefährdend ist, geht sie oft mit negativen Auswirkungen auf das Selbstbewusstsein und die Lebensqualität der Betroffenen einher.

Bei der androgenetischen Alopezie kommt es zu einer Verkürzung der Wachstumsphase des Haares und damit zu einer Verminderung der Haarschaftdicke. Dieser als Miniaturisierung bezeichnete Prozess wandelt die dicken Terminalhaare des Kopfes in dünne Flaumhaare (Vellushaare) um, wie sie den ganzen Körper bedecken. Am Ende dieses Prozesses wird das Haarwachstum vollständig eingestellt. Gesunde Kopfhaare wachsen kontinuierlich für mehrere Jahre, bevor der Haarfollikel in eine mehrmonatige Ruhephase (Telogen) eintritt. Beim Wiedereintritt in die Wachstumsphase (Anagen) bildet sich ein neuer Haarschaft, wobei der alte ausfällt. Der Verlust von 50-100 Haaren am Tag ist somit völlig normal.

Eine entscheidende Rolle bei der Entstehung der androgenetischen Alopezie spielen männliche Geschlechtshormone (Androgene). Hierzu gehören sowohl Testosteron als auch sein Umwandlungsprodukt Dihydrotestosteron (DHT). Letzteres hat aufgrund seiner stärkeren Affinität zum Androgenrezeptor eine stärkere androgene Wirkung als Testosteron selbst und spielt deshalb ebenfalls eine große Rolle für Körperfunktionen. In den meisten Fällen haben Männer mit androgenetischem Haarausfall zwar normale Androgenspiegel, aber spezielle Zellen im Haarfollikel, die das Wachstum des Haares steuern, reagieren bei ihnen empfindlicher auf Dihydrotestosteron (DHT) (Mowszowicz I et al. J Clin Endocrinol Metab 56, Issue 6, 1 June 1983, Pages 1209-1213).

Die Umwandlung von Testosteron in Dihydrotestosteron wird durch das Enzym $5\text{-}\alpha$-Reduktase katalysiert. Dieses Enzym existiert in drei Isoformen, die sich strukturell sowie in ihrer Gewebeverteilung deutlich voneinander unterscheiden.

$5\alpha$-Reduktase Typ I kommt vor allem in der Haut einschließlich der Kopfhaut vor. Die Aktivität der $5\text{-}\alpha$-Reduktase ist vor allem in der Dermalpapille des Haarfollikels nachweisbar, in der die Steuerungszellen für das Haarwachstum liegen und die somit das primäre Zielorgan für die Wirkung von Dihydrotestosteron darstellt.

$5\alpha$-Reduktase Typ II kommt vor allem in Prostata und Hoden vor.

Inzwischen ist noch ein dritter Typ der $5\text{-}\alpha$-Reduktase entdeckt worden, der ubiquitär im Körper vorkommt, z.B. in Herz, Gehirn, Magen, Muskelgewebe, Hoden und Haut. Die Funktionen sind noch unklar (Godoy A et al. Prostate 2011 July 71(10): 1033-1046; Yamana K et al. Horm Mol Biol Clin Investig. 2010 Aug 1;2(3):293-9).

Eine starke Hemmung der $5\alpha$-Reduktase vom Typ II führt zu einer deutlichen Reduktion der systemischen Konzentrationen von Androgenen, insbesondere DHT, was mit negativen Wirkungen wie verminderter Libido, erektiler Dysfunktion und Depression verbunden ist (Fertig et al. (2017) Dermatol Online J 23 (11).

Aktuell existieren 2 zugelassene Arzneimittel zur Behandlung der androgenetischen Alopezie: Minoxidil und Finasterid.

Minoxidil wurde ursprünglich als blutdrucksenkendes Mittel entwickelt und wird heute zur äusserlichen Behandlung des androgenetischen Haarausfalls bei Frauen und Männern eingesetzt. Es muss mehrmals täglich lokal aufgetragen werden. Die Wirkungsweise ist nicht endgültig geklärt; man geht davon aus, dass Minoxidil aufgrund seiner blutdrucksenkenden Wirkung die Kapillaren erweitert und somit die Durchblutung fördert. Zusätzlich kann Minoxidil den Haarausfall aufhalten, indem es die Ruhephase (Telogenphase) des Haarzyklus verkürzt. Dadurch wird die Wachstumsphase (Anagenphase) schneller erreicht und das Wachstum neuer Haare angeregt. Zu den häufigsten unerwünschten Wirkungen gehören leichte Ekzeme der Kopfhaut..

Finasterid ist ein selektiver Inhibitor der Steroid-5-$\alpha$-Reduktase der als Arzneistoff bei benigner Prostatahyperplasie (BPH) und bei androgenbedingtem Haarausfall (androgenetische Alopezie, AGA) zugelassen ist. Ein weiterer Inhibitor des Enzyms - Dutasterid - ist bis jetzt nur zur Behandlung der BPH zugelassen

Allerdings ist die Wirkung von Finasterid auf den Typ I der 5-$\alpha$-Reduktase erheblich geringer als auf den Typ II (Azzouni et al. Advances in Urology, 2012;2012:530121).

Aufgrund der starken Aktivität von Finasterid auf den Typ II der 5-$\alpha$ Reduktase kommt es zu einer Beeinflussung des männlichen Hormonhaushalts in Form reduzierter Spiegel an dem Androgen DHT und damit zu Nebenwirkungen wie verminderter Libido oder sexuellen Funktionsstörungen bis hin zu langanhaltender Impotenz auch nach Absetzen des Medikaments. Aus diesem Grund wird die medikamentöse Therapie der AA häufig abgebrochen oder gar nicht erst begonnen Liu et al. (2016) J Sex Med 13(9) 1297-310; Kiguradze et al. (2017) PeerJ, DOI 10.7717/peerj.3020;

Neben den zugelassenen Arzneimitteln Finasterid sind auch Zubereitungen mit pflanzlichen Extrakten mit 5-$\alpha$ Reduktase inhibierender Wirkung zur Behandlung der Androgenetischen Alopezie bzw. allg. zur Verbesserung des Haarwachstums beschrieben.

So konnte Rossi et al. in einer vergleichenden Studie mit Finasterid zeigen, dass ein Extrakt aus Sägepalmfrüchten (Serenoa repens) eine Verbesserung der androgenetischen Alopezie bewirken kann (Int J Immunopathol Pharmacol. 2012 Oct-Dec;25(4):1167-73).

In einer placebo-kontrollierten Pilotstudie mit Sägepalm-Extrakt konnte bei 6 von 10 Probanden eine Verbesserung der AGA nach Einnahme von Sägepalm-Extrakt und Beta-Sitosterol über 26 Wochen gemessen werden (Prager N et al. J Altern Complement Med. 2002 Apr;8(2):143-52).

Kwon et al. konnten zeigen, dass das in Grüntee-Extrakt enthaltene Epigallocatechin-3-gallate (EGCG) das Haarwachstum in einer ex-vivo Kultur von Haarfollikeln stimuliert (Kwon et al. Phytomedicine 2007 Aug; Vol14, Issues 7-8, p551-555)

Cho et al. konnte zeigen, dass die die Gabe von Kürbiskernöl bei Probanden mit AGA nach 24 Wochen zu einer signifikanten Verbesserung des Erscheinungsbildes und der Haaranzahl führt (Cho et al. Evidence-Based Complementary and Alternative Medicine, Volume 2014, Article ID 549721).

Des Weiteren gibt es eine Reihe von Patentschriften, die unterschiedliche Zusammensetzungen mit verschiedenen Pflanzenextrakten zur Behandlung von Haarausfall offenbaren.

In der DE 10 2004 039 983 werden Zusammensetzungen offenbart, die Bockshornkleesamenpulver und Leinsamenextrakt enthalten und die insbesondere zur Wiederbelebung und/oder zum Anregen und/oder zum Verstärken des Haarwuchses vorgesehen ist.

In der WO2007/034323 werden Zusammensetzungen offenbart, die Bockshornkleesamen-Extrakt, Leinsamenextrakt und Sägepalm-Extrakt enthalten, zur Behandlung der androgenetischen Allopezie.

In der US 20060009430 werden Zusammensetzung zur Verhinderung oder Behandlung der schädlichen Wirkungen von Dihydrotestosterone (DHT) insbesondere zur Behandlung von Alopezie und Prostatahyperplasie umfassend u.a. Sägepalmen-Extrakt, Kürbiskernextrakt und Beta-Sitosterol und Quercetin offenbart.

WO00/256269 offenbart natürliche Zusammensetzungen zur Behandlung von Haarausfall beim Mann, die eine Kombination aus Sägepalm-Extrakt, Pygeum Africanum-Extrakt und Brennnessel-Extrakt sowie zusätzlich Grüntee-Extrakt enthalten.

Ob und in welchem Maße die oben genannten Pflanzenextrakte eine unterschiedliche Inhibierung der beiden Subtypen der 5-alpha-Reduktase bewirken, wurde in den oben genannten Zitierungen nicht untersucht.

Daher ist es wünschenswert, Zusammensetzungen mit pflanzlichen Extrakten und Ölen bereitzustellen, die eine möglichst ausgewogene inhibierende Wirkung auf die beiden Subtypen der 5 alpha Reduktase, vom Subtyp I und vom Subtyp II aufweisen und die somit zur Verbesserung und/oder Stärkung des Haarwachstums bei Androgenetischer Alopezie eingesetzt werden können ohne dass mit unerwünschten Nebenwirkungen zu rechnen ist

Dies gilt insbesondere, wenn die Zusammensetzung unter anderen auch für die Verwendung als Nahrungsergänzungsmittel oder diätetisches Lebensmittel geeignet sein soll.

## Aufgabe der Erfindung

Es soll eine oral einzunehmende Zusammensetzung bereit gestellt werden, die insbesondere als Nahrungsergänzungsmittel oder Diätetisches Lebensmittel eingesetzt werden kann und auf nutritivem Weg - über Verabreichung von Pflanzenextrakten bzw. -ölen - zur Verminderung von Haarausfall und zu einer Verbesserung des Haarwachstums bei Androgenetischer Alopezie (AA) führt. Dabei sollen die erheblichen Nebenwirkungen der gegenwärtig verfügbaren pharmakologischen Therapie vermieden werden.

## Zusammenfassung der Erfindung

Die vorliegende Erfindung löst das Problem durch die Bereitstellung einer oral einzunehmenden Zusammensetzung wobei die Zusammensetzung bezogen auf eine Tagesdosis der Zusammensetzung eine Kombination aus 100

- 500 mg Kakao-Extrakt, 100 - 500 mg Leinsamen-Extrakt und 100 - 1000 mg Kürbiskernöl umfasst.

Die verwendeten Pflanzenextrakte und -öle der vorliegenden Erfindung bzw. der erfindungsgemäßen Zusammensetzung weisen überraschenderweise eine ausgewogene Wirkung auf die beiden Typen (Typ I und Typ II) der 5 alpha Reduktase auf, wobei mit ausgewogener Wirkung eine etwas gleich starke Inhibierung der 5 alpha Reduktase vom Typ I und Typ II gemeint ist.

Insbesondere wurde überraschenderweise gefunden, dass Kakao Extrakt, Leinsamen-Extrakt, Kürbiskernöl, Grüntee-Extrakt und Sägepalmfrüchte-Öl eine ausgewogene Wirkung auf die beiden Typen (Typ I und Typ II) der 5 alpha Reduktase aufweisen.

Ferner wurde überraschenderweise gefunden, dass die inhibierende Wirkung der in der erfindungsgemäßen Zusammensetzung verwendeten Pflanzenextrakte auf die 5 alpha Reduktase vom Typ I in etwa gleich oder nur etwas geringer ausfällt als die inhibierende Wirkung der verwendeten Pflanzenextrakte auf die 5 alpha Reduktase vom Typ II.

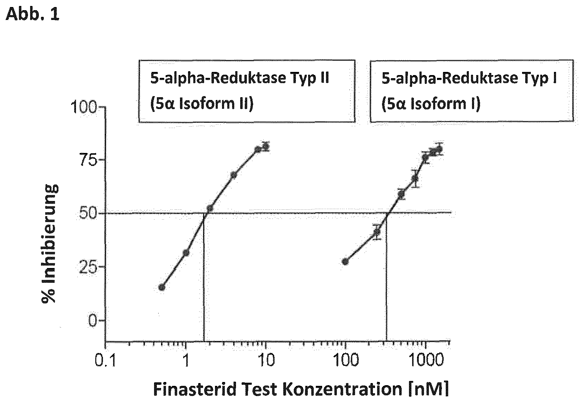

Im Gegensatz dazu konnte bestätigt werden werden, dass die inhibierende Wirkung von Finasterid auf den Typ I der $5\alpha$-Reduktase erheblich geringer ist als auf den Typ II. Um die Aktivität des Enzyms um 50% zu hemmen, ist beim Typ I die ca. 200fache Konzentration notwendig (Abbildung 1).

Durch die erfindungsgemäße Zusammensetzung soll ein übermäßig starkes Absinken der systemischen DHT-Konzentration, hervorgerufen durch eine zu starke Inhibierung der 5-alpha Reduktase II vermieden werden, wodurch die oben beschriebenen Nebenwirkungen, die insbesondere in Zusammenhang mit Finasterid und Dutasterid diskutiert werden, ebenfalls vermieden werden.

Durch die zu erwartenden geringen Nebenwirkungen eignet sich die erfindungsgemäße Zusammensetzung insbesondere als Nahrungsergänzungsmittel oder als Diätetisches Lebensmittel zur Verminderung von Haarausfall und zur Verbesserung des Haarwuchses bei Androgenetischer Alopezie oder als pharmazeutische Mittel zur Verwendung der Behandlung der androgenetischen Alopezie.

**Beschreibung der Abbildungen:**

Abbildung 1 zeigt die Inhibierung der 5-alpha Reduktase vom Typ I ($5\alpha$ Isoform I) und der 5-alpha Reduktase vom Typ II ($5\alpha$ Isoform II) durch Finasterid. Der $IC_{50}$-Wert (Finasterid Konzentration bei der eine 50%ige Hemmung erreicht wird) für die 5 alpha Reduktase vom Typ I liegt bei 375,6 $\pm$ 29,0 [nM] Finasterid, während der $IC_{50}$-Wert für die 5 alpha Reduktase vom Typ II bei 1,89 $\pm$ 0,02 [nM] Finasterid liegt.

Die gemessene Wirkung von Finasterid auf die 5-alpha Reduktase vom Typ II ($5\alpha$ Isoform II) ist demnach ca. 200 fach stärker als die Wirkung auf die 5-alpha Reduktase vom Typ I ($5\alpha$ Isoform I).

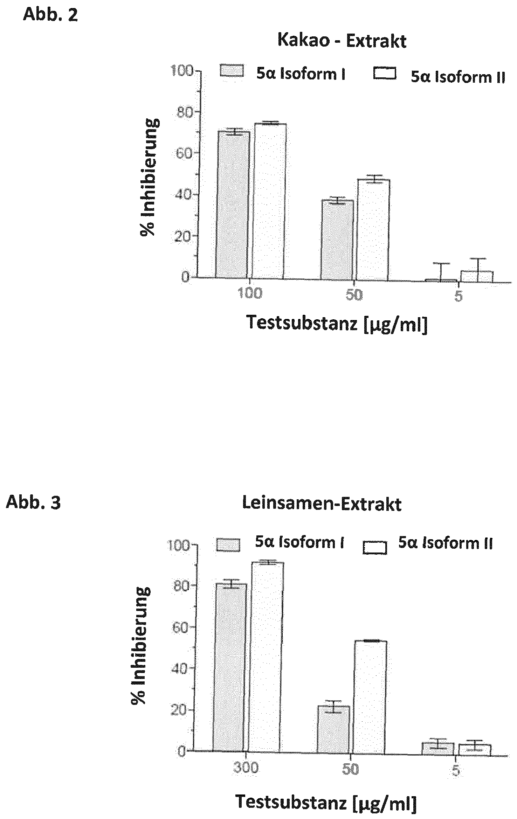

Abbildungen 2-9 zeigen das inhibitorische Potential verschiedener Pflanzenextrakte oder Pflanzenöle auf die 5-alpha Reduktase Isoformen vom Typ I und Typ II in Prozent für jeweils unterschiedliche Konzentrationen an eingesetzter Testsubstanz.

**Detaillierte Beschreibung der Erfindung**

In einer Ausführungsform umfasst die Erfindung eine oral einzunehmende Zusammensetzung wobei die Zusammensetzung bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl umfasst.

In einer weiteren Ausführungsform umfasst die Erfindung eine oral einzunehmende Zusammensetzung wobei die Zusammensetzung bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl und zusätzlich 2- 100 $\mu$g Vitamin D und/oder 2-20 mg Zink umfasst.

In einer weiteren Ausführungsform umfasst die Erfindung eine oral einzunehmende Zusammensetzung wobei das Produkt bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl und zusätzlich 100 - 500 mg Grüntee-Extrakt und/oder 100 -500 mg Sägepalmfrüchte-Öl umfasst.

In einer weiteren Ausführungsform umfasst die Erfindung eine oral einzunehmende Zusammensetzung, wobei die Zusammensetzung bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl, 2- 100 µg Vitamin D und/oder 2-20 mg Zink und zusätzlich 100 - 500 mg Grüntee-Extrakt und/oder 100 -500 mg Sägepalmfrüchte-Öl umfasst.

In einer weiteren Ausführungsform umfasst die Erfindung eine oral einzunehmende Zusammensetzung wobei das Produkt bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl und zusätzlich 100 - 400 mg Curcumin/Curcuma-Extrakt und/oder 0,5- 6 mg Lycopin umfasst.

In einer weiteren Ausführungsform umfasst die Erfindung eine oral einzunehmende Zusammensetzung wobei die Zusammensetzung bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernölund 2- 100 µg Vitamin D und/oder 2-20 mg Zink und zusätzlich 100 - 400 mg Curcumin/Curcuma-Extrakt und/oder 0,5- 6 mg Lycopin umfasst.

In einer weiteren Ausführungsform umfasst die Erfindung eine oral einzunehmende Zusammensetzung wobei die Zusammensetzung bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl und 100 - 500 mg Grüntee-Extrakt und/oder 100 -500 mg Sägepalmfrüchte-Öl und zusätzlich 100 - 400 mg Curcumin/Curcuma-Extrakt und/oder 0,5- 6 mg Lycopin umfasst.

In einer weiteren Ausführungsform umfasst die Erfindung eine oral einzunehmende Zusammensetzung wobei die Zusammensetzung bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl, 2- 100 µg Vitamin D und/oder 2-20 mg Zink und 100 - 500 mg Grüntee-Extrakt und/oder 100 -500 mg Sägepalmfrüchte-Öl und zusätzlich 100 - 400 mg Curcumin/Curcuma-Extrakt und/oder 0,5- 6 mg Lycopin umfasst.

Für den Fachmann ist es ersichtlich, dass die oben beschriebenen erfindungsgemäßen Zusammensetzungen insbesondere durch die Menge an Inhaltsstoffen definiert wird, die pro Tag oder als Tagesdosis eingenommen wird.

Die Zusammensetzung im Sinne der Erfindung kann aus einer, zweier oder mehrerer gleicher oder unterschiedlicher Darreichungsformen bestehen, die die erfindungsgemäße Kombination mit der jeweiligen Tagesdosis an Pflanzenextrakten und -ölen enthalten.

Geeignete Darreichungsformen im Sinne der Erfindung sind alle Darreichungsformen, die zur oralen Einnahme bestimmt, geeignet oder verwendbar sind.

Geeignete Darreichungsformen sind beispielsweise Kapseln, wie z.B. Weichgelatinekapsel, Lösungen, Tabletten, wie z.B. Filmtabletten, Pulver, Emulsionen, Granulate, Riegel, Toffee oder andere kaubare, schluckbare oder trinkbare Formen.

Die Verwendung unterschiedlicher Darreichungsformen ist insbesondere dann sinnvoll, wenn sich erfindungsgemäße Kombination an Pflanzenextrakten und -ölen pro Tagesdosis schlecht oder nur mit großem Aufwand untereinander mischen lassen.

Auch kann es sinnvoll sein die Tagesdosis der verendeten erfindungsgemäßen Kombination an Pflanzenextrakten und -ölen auf mehrere Teile einer bestimmten Darreichungsform aufzuteilen, weil die Bereitstellung der gesamten Tagesdosis der erfindungsgemäßen Kombination an Pflanzenextrakten und -ölen in einer einzigen Darreichungsform, beispielsweise einer Tablette aufgrund der Menge zu einer zu großen oder schwere Darreichungsform führt, die sich aufgrund dessen nur schwer verabreichen läßt.

Bevorzug liegt die erfindungsgemäße Zusammensetzung, insbesondere, wenn sie aus mehreren Teilen oder unterschiedlichen Darreichungsformen besteht innerhalb eines Gebindes, eines Kits oder einer Produkt- oder Verpackungseinheit vor.

Unter dem Begriff "oral einzunehmende Zusammensetzung" wird im Sinne dieser Erfindung verstanden, dass die Zusammensetzung zur oralen Verwendung insbesondere zum Verzehr geeignet oder bestimmt ist.

Unter dem Begriff "Tagesdosis" ist die Menge an Extrakten oder Inhaltsstoffen gemeint, die pro Tag oder innerhalb eines Tages oder innerhalb von 24 Stunden eingenommen werden soll. Die erforderliche Tagesdosis kann in Form einer oder mehrerer gleicher oder verschiedener Darreichungsformen eingenommen werden.

Der Begriff "Androgenetische Alopezie" hat die dem Fachmann bekannte Bedeutung und ist gleichbedeutend oder umfasst u.a. erblich bedingter Haarausfall, androgenetischer Haarausfall, Alopecia androgenetica oder AGA.

Unter dem Unter dem Begriff "Extrakt" werden im Sinne dieser Erfindung konzentrierte, gegebenenfalls auf einen bestimmten Gehalt an Inhaltsstoffen eingestellte, Auszüge aus Früchten oder anderen Bestandteilen von Pflanzen von flüssiger, zähflüssiger, öliger oder trockener Beschaffenheit verstanden. Extrakte auf pflanzlicher Basis können beispielsweise unter Verwendung von Alkohol oder Wasser gewonnen werden. Unter dem Begriff "Extrakt" werden im Sinne dieser Erfindung ebenfalls gereinigte Extrakte oder Extraktfraktionen verstanden. Unter dem Begriff "Extrakt aus" wird im Sinne dieser Erfindung verstanden, dass der Extrakt aus der entsprechenden Pflanze oder Pflanzenteilen erhältlich oder hergestellt ist.

Der Kakao-Extrakt umfasst Extrakte, die aus den Bohnen der Pflanze Theobroma Cacao gewonnen werden. Kakao-Extrakt oder Kakao-Bohnen-Extrakt wird hierbei synonym verwendet. Kakao-Extrakte können mit üblichen Extraktionsmitteln wie z.B. Ethanol-Wasser-Gemischen gewonnen und konzentriert werden. Kakao-Extrakt ist kommerziell beispielsweise erhältlich von der Firma Steffes Ingredients GmbH.

Der Leinsamenextrakt umfasst Extrakte aus den Samen der Pflanze Linum usitatissimun L., die mittels herkömmlicher Verfahren hergestellt werden. Hierzu gehört beispielsweise die Extraktion mit Ethanol-Wasser-Gemischen. Leinsamenextrakt ist kommerziell beispielsweise erhältlich von der Firma Steffes Ingredients GmbH.

Kürbiskernöl umfasst ein mittels herkömmlicher Verfahren beispielsweise durch mechanische Verfahren wie Pressen und ggf. Filtrieren hergestelltes Öl aus der Pflanze Cucurbita pepo L., Kürbiskernöl ist kommerziell beispielsweise erhältlich von der Firma Gustav Heess GmbH.

Zink, umfasst Zink und alle pharmazeutisch üblichen Salze und Derivate davon beispielsweise in Form von Zinkgluconat, Zinkacetat, Zinkoxid oder Zinkstearat. In einer bevorzugten Ausführungsform liegt Zink in Form von Zinkgluconat vor. In einer weiteren bevorzugten Ausführungsform liegt Zink in Form von Zinkoxid vor. Zink ist an einer Vielzahl von körpereigenen Vorgängen beteiligt und spielt unter anderem eine wichtige Rolle beim Haarwachstum.

Vitamin D umfasst insbesondere Vitamin $D_3$ auch bezeichnet als Cholecalciferol, Colecalciferol oder Calciol.

Curcumin umfasst (1$E$,6$E$)-1,7-Bis(4-hydroxy-3-methoxyphenyl)-hepta-1,6-dien-3,5-dion, Curcumagelb, Diferuloylmethan oder E 100 und alle isomeren und tautomeren Formen davon und kann sowohl traditionell aus Pflanzen der Pflanzengattung Curcuma hergestellt werden oder synthetisch mit an sich bekannten Methoden.

Curcuma-Extrakt umfasst Extrakte der Pflanze Curcuma longa, die mittels herkömmlicher Verfahren hergestellt werden und die im wesentlichen Curcumin enthalten.

Grüntee-Extrakt umfasst Extrakte aus Camellia sinensis, die mittels herkömmlicher Verfahren hergestellt werden. Hierzu gehört beispielsweise die Extraktion mit Ethanol-Wasser-Gemischen. Grüntee-Extrakt ist kommerziell beispielsweise erhältlich von der Firma Denk Ingredients GmbH.

Sägepalmfrüchte-Öl, umfasst ölige Extrakte aus den Früchten der Sägepalme (Serenoa repens), die mittels herkömmlicher Verfahren hergestellt werden. Beispielsweise in Form von Extrakten mit Ethanol als Auszugsmittel. Sägepalmfrüchte-Extrakt ist kommerziell beispielsweise erhältlich von der Firma Aceto Health Ingredients GmbH.

Lycopin umfasst sowohl aus Tomaten oder anderen geeigneten Pflanzen gewonnenes oder extrahiertes Lycopin als auch mit an sich bekannten synthetischen Methoden hergestelltes Lycopin. Lycopin ist auch unter dem Namen Lycopen oder Leukopin bekannt.

Erfindungsgemäß liegt die Zusammensetzung die pro Tag eingenommen wird in Form einer, zweier oder mehrerer gleicher oder unterschiedlicher Darreichungsformen vor.

In einer Ausführungsform liegt die Tagesdosis der erfindungsgemäßen Zusammensetzung in Form einer einzigen Darreichungsform vor. Die jeweilige Darreichungsform der Zusammensetzung kann dabei in Form einer oder mehrerer Teile vorliegen beispielsweise in Form einer oder mehrerer Kapseln (beispielsweise Weichgelatinekapsel), einer oder mehrerer Lösungen, oder einer oder mehrerer Tabletten (beispielsweise Filmtabletten), einer oder mehrerer Pulver, einer oder mehrerer Emulsionen, einer oder mehrerer Granulate, einer oder mehrerer Riegel, einer oder mehrerer Toffees oder in einer oder mehrerer anderer kaubarer, schluckbarer oder trinkbarer Formen vorliegen.

In einer weiteren Ausführungsform liegt die erfindungsgemäße Zusammensetzung in einer einzigen Darreichungsform vor, wobei die gesamte Tagesdosis der verwendeten Extrakte oder Inhaltsstoffe in Form eines einzigen Teils dieser Darreichungsform vorliegt, beispielsweise in Form einer Kapsel, einer Lösung, einer Tablette, eines Pulvers, einer Emulsion, eines Granulats, eines Riegels, eines Toffees oder einer anderen kaubaren, schluckbaren oder trinkbaren Form.

In einer weiteren bevorzugten Ausführungsform liegt die erfindungsgemäße Zusammensetzung in einer einzigen Darreichungsform vor, wobei die gesamte Tagesdosis der verwendeten Extrakte oder Inhaltsstoffe in Form von zwei oder drei oder vier oder mehreren Teilen dieser Darreichungsform vorliegt, beispielsweise in Form von zwei oder drei oder vier oder mehrerer Kapseln, zwei oder drei oder vier oder mehrerer Lösungen, zwei oder drei oder vier oder mehrerer Tabletten, zwei oder drei oder vier oder mehrerer Pulver, zwei oder drei oder vier oder mehrerer Emulsionen, zwei oder drei oder vier oder mehrerer Granulate, zwei oder drei oder vier oder mehrerer Riegel, zwei oder drei oder vier oder mehrerer Toffees oder zwei oder drei oder vier oder mehrerer anderer kaubarer, schluckbarer oder trinkbarer Formen und wobei die zwei oder drei oder vier oder mehreren Teilen der jeweiligen Darreichungsform die gleiche Menge der verwendeten Extrakte oder Inhaltsstoffe bzw. die gleichen Komponenten der Zusammensetzung aufweisen.

In einer weiteren besonders bevorzugten Ausführungsform liegt die Zusammensetzung in Form zweier oder mehrerer unterschiedlicher Darreichungsformen vor, wobei die gesamte Tagesdosis der verwendeten Extrakte oder Inhaltsstoffe in Form zweier oder mehrerer unterschiedlicher Darreichungsformen vorliegt und wobei die unterschiedlichen Darreichungsformen unterschiedliche Komponenten der Zusammensetzung aufweisen. Die jeweiligen Darreichungsformen der Zusammensetzung können dabei in Form einer, zweier, dreier oder mehrerer Teile vorliegen, beispielsweise in Form einer, zweier, dreier oder mehrerer Kapseln, einer , zweier, dreier oder mehrerer Lösungen, einer , zweier, dreier oder mehrerer Tabletten, einer , zweier, dreier oder mehrerer Pulver, einer, zweier, dreier oder mehrerer Emulsionen, einer, zweier, dreier oder mehrerer Granulate, einer , zweier, dreier oder mehrerer Riegel, einer , zweier, dreier oder mehrerer Toffees oder einer , zweier, dreier oder mehrerer anderer kaubarer, schluckbarer oder trinkbarer Formen, mit der Maßgabe, dass die Zusammensetzung mindestens zwei unterschiedliche Darreichungsformen aufweist, wie beispielsweise in Form einer Tablette und einer Kapsel oder beispielsweise in Form einer Trinkampule und einer Kapsel oder beispielsweise in Form zweier Tabletten und einer Kapsel.

In einer besonders bevorzugten Ausführungsform liegt die erfindungsgemäße oral einzunehmende Zusammensetzung in Form zweier unterschiedlicher Darreichungsformen vor, mit der Maßgabe, dass eine der beiden Darreichungsformen eine Kapsel (z.B. Weichgelatinekapsel) ist und 100 - 1000 mg Kürbiskernöl, bevorzugt 200- 700 mg Kürbiskernöl, besonders bevorzugt 250 - 400 mg Kürbiskernöl enthält und die andere Darreichungsform ausgewählt ist aus einer oder mehrerer Lösungen, oder einer oder mehrerer Tabletten (beispielsweise Filmtabletten), einer oder mehrerer Pulver, einer oder mehrerer Emulsionen, einer oder mehrerer Granulate, einer oder mehrerer Riegel, einer oder mehrerer Toffees oder einer oder mehrerer anderer kaubarer, schluckbarer oder trinkbarer Formen und wobei dieses Darreichungsformen 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200- 400 mg Leinsamenextrakt, besonders bevorzugt 250 - 350 mg Leinsamenextrakt enthalten.

In einer ganz besonders bevorzugten Ausführungsform liegt die erfindungsgemäße oral einzunehmende Zusammensetzung in Form einer Kapsel (beispielsweise Weichgelatinekapsel) und einer Tablette vor, wobei die Kapsel 100 - 1000 mg Kürbiskernöl, bevorzugt 200- 700 mg Kürbiskernöl, besonders bevorzugt 250 - 400 mg Kürbiskernöl enthält und die Tablette 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200- 400 mg Leinsamenextrakt, besonders bevorzugt 250 - 350 mg Leinsamenextrakt enthält.

In einer weiteren bevorzugten Ausführungsform liegt die erfindungsgemäße oral einzunehmende Zusammensetzung in Form einer Kapsel und einer Lösung (z. B. in Form einer Trinkampule) vor, wobei die Kapsel 100 - 1000 mg Kürbiskernöl, bevorzugt 200- 700 mg Kürbiskernöl, besonders bevorzugt 250 - 400 mg Kürbiskernöl enthält und die Lösung 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200- 400 mg Leinsamenextrakt, besonders bevorzugt 250 - 350 mg Leinsamenextrakt enthält.

In einer weiteren bevorzugten Ausführungsform liegt die erfindungsgemäße oral einzunehmende Zusammensetzung in Form einer Kapsel (beispielsweise Weichgelatinekapsel) und zweier Tabletten vor, wobei die Kapsel 100 - 1000 mg Kürbiskernöl, bevorzugt 200- 700 mg Kürbiskernöl, besonders bevorzugt 250 - 400 mg Kürbiskernöl enthält und die beiden Tabletten zusammen insgesamt zu gleichen Teilen 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200- 400 mg Leinsamenextrakt, besonders bevorzugt 250 - 350 mg Leinsamenextrakt enthalten.

In weiteren Ausführungsformen kann die Zusammensetzung wenigstens einen Zusatz, vorzugsweise mehrere Zusätze, zur Verbesserung der Bioverfügbarkeit, zur Verbesserung der Textur, zur Verbesserung der Löslichkeit und/oder zur Verbesserung der Lösegeschwindigkeit, Sprengmittel, Stoffe die die Haltbarkeit verbessern, geschmacksmaskierende Stoffe, geschmacksverbessernde Stoffe, Stoffe zur Erhöhung oder Senkung der Viskosität, Stabilisatoren, Farbstoffe, Füllstoffe, Formentrennmittel und/oder Zerfallsbeschleuniger aufweisen.

Die erfindungsgemäße Zusammensetzung kann Zusatzstoffe in einer Menge von 5 bis 99 Gew.%, oder bevorzugt 10 bis 99 Gew.%, insbesondere 20 bis 98 Gew.%, bezogen auf das Gesamtgewicht der Zusammensetzung aufweisen, welche aus der Herstellung der genannten oralen Darreichungsformen resultieren: Träger in flüssiger oder fester Form sowie pharmazeutisch zulässige Zusätze oder auch Zusatzstoffe, die für die Herstellung von Supplementen wie Nahrungsergänzungsmittel, ergänzende bilanzierte Diät, Diätikum bekannt sind.

Üblicherweise besteht die Trägergrundlage bei festen oralen Verabreichungsformen (Tablette, Kapsel, Dragee) aus üblichen, auch pharmazeutisch zulässigen; Tablettierhilfsstoffen wie Sprengmittel, Füllstoffe, Fließmittel, ggf. auch Lösehilfen wie Zitronensäure, Hydrogencarbonat (z. B. für Brausetabletten), Flüssigkeiten wie Wasser, (Frucht)Säfte, Kohlenhydrat-Protein-Mischungen (für Riegel) oder z. B. aus Gelatineweichkapseln, in die die Zusammensetzung inkorporiert wird. Besonders konzipierte Darreichungsformen sind hier nicht erforderlich, es werden die jeweils nach dem Stand der Technik bekannten Methoden und Produkte angewendet.

Die erfindungsgemäße Zusammensetzung bzw. die zuvor beschriebenen Ausführungsformen der Zusammensetzung können zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie verwendet werden. Ferner können die erfindungsgemäße Zusammensetzung bzw. die zuvor beschriebenen Ausführungsformen der Zusammensetzung zur Herstellung eines Mittels zur diätetischen Behandlung oder diätetischen Unterstützung einer Behandlung von Haarausfall und zur Verbesserung des Haarwuchses bei Androgenetischer Alopezie verwendet werden. Die erfindungsgemäße Zusammensetzung bzw. die zuvor beschriebenen Ausführungsformen der Zusammensetzung sind weiterhin geeignet als Mittel zur bilanzierten Ergänzung der Ernährung bei Menschen mit Androgenetischer Alopezie. Die erfindungsgemäße Zusammensetzung bzw. die zuvor beschriebenen Ausführungsformen der Zusammensetzung sind insbesondere geeignet als ergänzende bilanzierte Diät.

Der Begriff "diätetische Behandlung" ist in der EU-Richtlinie 1999/21/EG, die seit 01.01.2002 als 10. Verordnung zur Änderung der Diätverordnung in deutsches Recht umgesetzt worden ist, näher erläutert.

Die erfindungsgemäße Zusammensetzung bzw. die zuvor beschriebenen Ausführungsformen der Zusammensetzung können ferner als Nahrungsergänzungsmittel zur Verminderung von Haarausfall und zur Verbesserung des Haarwuchses bei androgenerischer Alopezie verwendet werden.

Die erfindungsgemäße Zusammensetzung bzw. die zuvor beschriebenen Ausführungsformen der Zusammensetzung können ferner als Nahrungsergänzungsmittel zur Verminderung von Haarausfall und zur Verbesserung des Haarwuchses bei Männern verwendet werden.

Die erfindungsgemäße Zusammensetzung bzw. die zuvor beschriebenen Ausführungsformen der Zusammensetzung können ferner als Nahrungsergänzungsmittel zur Verminderung von Haarausfall und zur Verbesserung des Haarwuchses bei Männer mit androgenetischer Alopezie verwendet werden.

Die erfindungsgemäße Zusammensetzung bzw. die zuvor beschriebenen Ausführungsformen der Zusammensetzung können ferner als pharmazeutische Zusammensetzung zur Verwendung bei der Behandlung der androgenetischen Alopezie eingesetzt werden.

Als vorteilhaft hat sich hierbei erwiesen, dass die erfindungsgemäße Zusammensetzung bzw. die erfindungsgemäße Kombination an Pflanzenextrakten eine ausgewogene Wirkung auf die beiden Typen (Typ I und Typ II) der 5 alpha Reduktase aufweisen. wobei mit ausgewogener Wirkung eine etwas gleich starke Inhibierung der 5 alpha Reduktase vom Typ I und Typ II durch den jeweiligen Pflanzenextrakt gemeint ist.

Insbesondere ist die inhibierende Wirkung der gewählten Pflanzenextrakte der erfindungsgemäßen Zusammensetzung bzw. die erfindungsgemäße Kombination an Pflanzenextrakten auf die 5-alpha Reduktase I durch den jeweils gewählten Pflanzenextrakt entweder stärker, gleich oder maximal 2,5 x schwächer als die inhibierende Wirkung auf die 5-alpha Reduktase II bei gleicher Wirkstoffmenge des jeweils gewählten Pflanzenextraktes.

Gegenstand der Erfindung ist ferner die nicht-therapeutische Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl zur oralen Verabreichung pro Tag oder als Tagesdosis zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie oder zur Verminderung von Haarausfall und zur Verbesserung des Haarwuchses bei Androgenetischer Alopezie.

Ein weiter Gegenstand der Erfindung ist ferner die nicht-therapeutische Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl und zusätzlich 2- 100 μg Vitamin D und/oder 2-20 mg Zink zur oralen Verabreichung pro Tag oder als Tagesdosis zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie oder zur Verminderung von Haarausfall und zur Verbesserung des Haarwuchses bei Androgenetischer Alopezie.

Ein weiter Gegenstand der Erfindung ist ferner die nicht-therapeutische Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl. und zusätzlich 100 - 500 mg Grüntee-Extrakt und/oder 100 -500 mg Sägepalmfrüchte-Öl zur oralen Verabreichung pro Tag oder als Tagesdosis zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie oder zur Verminderung von Haarausfall und zur Verbesserung des Haarwuchses bei Androgenetischer Alopezie.

Ein weiter Gegenstand der Erfindung ist ferner die nicht-therapeutische Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl, , 2- 100 $\mu$g Vitamin D und/oder 2-20 mg Zink und zusätzlich 100 - 500 mg Grüntee-Extrakt und/oder 100 -500 mg Sägepalmfrüchte-Öl zur oralen Verabreichung pro Tag oder als Tagesdosis zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie oder zur Verminderung von Haarausfall und zur Verbesserung des Haarwuchses bei Androgenetischer Alopezie.

Ein weiter Gegenstand der Erfindung ist ferner die nicht-therapeutische Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl und zusätzlich 100 - 400 mg Curcumin/Curcuma-Extrakt und/oder 0,5-6 mg Lycopin zur oralen Verabreichung pro Tag oder als Tagesdosis zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie oder zur Verminderung von Haarausfall und zur Verbesserung des Haarwuchses bei Androgenetischer Alopezie.

Ein weiter Gegenstand der Erfindung ist ferner die nicht-therapeutische Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl und 2- 100 $\mu$g Vitamin D und/oder 2-20 mg Zink und zusätzlich 100 - 400 mg Curcumin/Curcuma-Extrakt und/oder 0,5- 6 mg Lycopin zur oralen Verabreichung pro Tag oder als Tagesdosis zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie oder zur Verminderung von Haarausfall und zur Verbesserung des Haarwuchses bei Androgenetischer Alopezie.

Ein weiter Gegenstand der Erfindung ist ferner die nicht-therapeutische Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl und 100 - 500 mg Grüntee-Extrakt und/oder 100 -500 mg Sägepalmfrüchte-Öl und zusätzlich 100 - 400 mg Curcumin/Curcuma-Extrakt und/oder 0,5- 6 mg Lycopin zur oralen Verabreichung pro Tag oder als Tagesdosis zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie oder zur Verminderung von Haarausfall und zur Verbesserung des Haarwuchses bei Androgenetischer Alopezie.

Ein weiter Gegenstand der Erfindung ist ferner die nicht-therapeutsche Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl, 2- 100 $\mu$g Vitamin D und/oder 2-20 mg Zink und 100 - 500 mg Grüntee-Extrakt und/oder 100 -500 mg Sägepalmfrüchte-Öl und zusätzlich 100 - 400 mg Curcumin/Curcuma-Extrakt und/oder 0,5- 6 mg Lycopin zur oralen Verabreichung pro Tag oder als Tagesdosis zur diätetischen Behandlung von Haarausfall und zur Verbesserung des Haarwuchses bei Androgenetischer Alopezie oder zur diätetischen Unterstützung einer Behandlung von Haarausfall und zur Verbesserung des Haarwuchses bei Androgenetischer Alopezie oder zur Verminderung von Haarausfall und zur Verbesserung des Haarwuchses bei Androgenetischer Alopezie.

Für den Fachmann ist es ersichtlich, dass die erfindungsgemäße nicht-therapeutische Verwendung der oben dargestellten Kombinationen die zur oralen Verabreichung pro Tag eingenommen wird nicht auf eine bestimmte Darreichungsform beschränkt ist, sondern mittels unterschiedlicher Darreichungsformen erfolgen kann.

So kann erfindungsgemäß die nicht-therapeutische Verwendung der oben dargestellten Kombinationen die zur oralen Verabreichung pro Tag eingenommen wird in Form einer, zweier oder mehrerer gleicher oder unterschiedlicher Darreichungsformen erfolgen.

In einer Ausführungsform kann erfindungsgemäß die nicht-therapeutische Verwendung der oben dargestellten Kombinationen die zur oralen Verabreichung pro Tag eingenommen wird in Form einer einzigen Darreichungsform erfolgen. Die jeweilige Darreichungsform der Zusammensetzung kann dabei in Form einer oder mehrerer Teile vorliegen beispielsweise in Form einer oder mehrerer Kapseln (beispielsweise Weichgelatinekapsel), einer oder mehrerer Lösungen, oder einer oder mehrerer Tabletten (beispielsweise Filmtabletten), einer oder mehrerer Pulver, einer oder mehrerer Emulsionen, einer oder mehrerer Granulate, einer oder mehrerer Riegel, einer oder mehrerer Toffees oder in einer oder mehrerer anderer kaubarer, schluckbarer oder trinkbarer Formen vorliegen.

In einer weiteren Ausführungsform kann erfindungsgemäß die nicht-therapeutische Verwendung der oben dargestellten Kombinationen die zur oralen Verabreichung pro Tag eingenommen wird in einer einzigen Darreichungsform erfolgen, wobei die gesamte Tagesdosis der verwendeten Extrakte oder Inhaltsstoffe in Form eines einzigen Teils dieser Darreichungsform vorliegt, beispielsweise in Form einer Kapsel, einer Lösung, einer Tablette, eines Pulvers, einer Emulsion, eines Granulats, eines Riegels, eines Toffees oder einer anderen kaubaren, schluckbaren oder trinkbaren Form.

In einer weiteren bevorzugten Ausführungsform kann erfindungsgemäß die nicht-therapeutische Verwendung der oben dargestellten Kombinationen die zur oralen Verabreichung pro Tag eingenommen wird in einer einzigen Darreichungsform erfolgen, wobei die gesamte Tagesdosis der verwendeten Extrakte oder Inhaltsstoffe in Form von zwei oder drei oder vier oder mehreren Teilen dieser Darreichungsform vorliegt, beispielsweise in Form von zwei oder drei oder vier oder mehrerer Kapseln, zwei oder drei oder vier oder mehrerer Lösungen, zwei oder drei oder vier oder mehrerer Tabletten, zwei oder drei oder vier oder mehrerer Pulver, zwei oder drei oder vier oder mehrerer Emulsionen, zwei oder drei oder vier oder mehrerer Granulate, zwei oder drei oder vier oder mehrerer Riegel, zwei oder drei oder vier oder mehrerer Toffees oder zwei oder drei oder vier oder mehrerer anderer kaubarer, schluckbarer oder trinkbarer Formen und wobei die zwei oder drei oder vier oder mehreren Teilen der jeweiligen Darreichungsform die gleiche Menge der verwendeten Extrakte oder Inhaltsstoffe bzw. die gleichen Komponenten der Zusammensetzung aufweisen.

In einer weiteren besonders bevorzugten Ausführungsform kann erfindungsgemäß die nicht-therapeutische Verwendung der oben dargestellten Kombinationen die zur oralen Verabreichung pro Tag eingenommen wird in Form zweier oder mehrerer unterschiedlicher Darreichungsformen erfolgen, wobei die gesamte Tagesdosis der verwendeten Extrakte oder Inhaltsstoffe in Form zweier oder mehrerer unterschiedlicher Darreichungsformen vorliegt und wobei die unterschiedlichen Darreichungsformen unterschiedliche Komponenten der Zusammensetzung aufweisen. Die jeweiligen Darreichungsformen der Zusammensetzung können dabei in Form einer, zweier, dreier oder mehrerer Teile vorliegen, beispielsweise in Form einer, zweier, dreier oder mehrerer Kapseln, einer , zweier, dreier oder mehrerer Lösungen, einer , zweier, dreier oder mehrerer Tabletten, einer , zweier, dreier oder mehrerer Pulver, einer , zweier, dreier oder mehrerer Emulsionen, einer, zweier, dreier oder mehrerer Granulate, einer, zweier, dreier oder mehrerer Riegel, einer, zweier, dreier oder mehrerer Toffees oder einer, zweier, dreier oder mehrerer anderer kaubarer, schluckbarer oder trinkbarer Formen, mit der Maßgabe, dass die Zusammensetzung mindestens zwei unterschiedliche Darreichungsformen aufweist, wie beispielsweise in Form einer Tablette und einer Kapsel oder beispielsweise in Form einer Trinkampule und einer Kapsel oder beispielsweise in Form zweier Tabletten und einer Kapsel.

In einer besonders bevorzugten Ausführungsform kann erfindungsgemäß die nicht-therapeutische Verwendung der oben dargestellten Kombinationen die zur oralen Verabreichung pro Tag eingenommen wird in Form zweier unterschiedlicher Darreichungsformen erfolgen, mit der Maßgabe, dass eine der beiden Darreichungsformen in Form einer Kapsel (z.B. Weichgelatinekapsel) vorliegt und 100 - 1000 mg Kürbiskernöl, bevorzugt 200- 700 mg Kürbiskernöl, besonders bevorzugt 250 - 400 mg Kürbiskernöl enthält und die andere Darreichungsform ausgewählt ist aus einer oder mehrerer Lösung(en), oder einer oder mehrerer Tablette(n) (beispielsweise Filmtablette(n)), einer oder mehrerer Pulver, einer oder mehrerer Emulsion(en), einem oder mehrerer Granulat(e), einer oder mehrerer Riegel, einer oder mehrerer Toffees oder einer oder mehrerer anderer kaubarer, schluckbarer oder trinkbarer Form(en) und wobei dieses Darreichungsformen 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200- 400 mg Leinsamenextrakt, besonders bevorzugt 250 - 350 mg Leinsamenextrakt enthalten.

In einer ganz besonders bevorzugten Ausführungsform kann erfindungsgemäß die nicht-therapeutische Verwendung der oben dargestellten Kombinationen die zur oralen Verabreichung pro Tag eingenommen wird in Form einer Kapsel (beispielsweise Weichgelatinekapsel) und einer Tablette erfolgen, wobei die Kapsel 100 - 1000 mg Kürbiskernöl, bevorzugt 200- 700 mg Kürbiskernöl, besonders bevorzugt 250 - 400 mg Kürbiskernöl enthält und die Tablette 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200- 400 mg Leinsamenextrakt, besonders bevorzugt 250 - 350 mg Leinsamenextrakt enthält.

In einer weiteren bevorzugten Ausführungsform kann erfindungsgemäß die nicht-therapeutische Verwendung der oben dargestellten Kombinationen die zur oralen Verabreichung pro Tag eingenommen wird in Form einer Kapsel und einer Lösung (z. B. in Form einer Trinkampule) erfolgen, wobei die Kapsel 100 - 1000 mg Kürbiskernöl, bevorzugt 200- 700 mg Kürbiskernöl, besonders bevorzugt 250 - 400 mg Kürbiskernöl enthält und die Lösung 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200- 400 mg Leinsamenextrakt, besonders bevorzugt 250 - 350 mg Leinsamenextrakt enthält.

In einer weiteren bevorzugten Ausführungsform kann erfindungsgemäß die nicht-therapeutische Verwendung der oben dargestellten Kombinationen die zur oralen Verabreichung pro Tag eingenommen wird in Form einer Kapsel (beispielsweise Weichgelatinekapsel) und zweier Tabletten erfolgen, wobei die Kapsel 100 - 1000 mg Kürbiskernöl, bevorzugt 200- 700 mg Kürbiskernöl, besonders bevorzugt 250 - 400 mg Kürbiskernöl enthält und die beiden Tabletten zusammen insgesamt zu gleichen Teilen 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200- 400 mg Leinsamenextrakt, besonders bevorzugt 250 - 350 mg Leinsamenextrakt enthalten.

Gegenstand der Erfindung ist ferner eine pharmazeutische Zusammensetzung wie zuvor in den Ausführungsbeispielen beschrieben zur Verwendung bei der Behandlung der androgenetischen Alopezie.

Eine bevorzugte Ausführungsform ist eine pharmazeutische Zusammensetzung wobei die Zusammensetzung bezogen auf eine Tagesdosis der Zusammensetzung eine Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl umfasst zur Verwendung bei der Behandlung der androgenetischen Alopezie.

Untersuchung verschiedener Extrakte pflanzlichen Ursprungs hinsichtlich der der Inhibierung der 5-$\alpha$-Reduktase Typ I and Typ II *in vitro* mit Hilfe eines Zell-freien Assay.

Material und Methoden:

Wichtigsten Apparate: LC-HRMS Instrument: Accela U-HPLC Pumpe und ein Accela Open auto sampler (Thermo Fisher Scientific) verbunden mit einem Q-Exactive mass spectrometer (Orbitrap™) ausgestattet mit einem beheiztem Electrospray Interface; Datenverarbeitung mit der Standard Software Xcalibur 2.2.

Getestete Pflanzenextrakte und Öle:

Tabelle 1:

| Testsubstanz | Hersteller/Lieferant |
| --- | --- |
| Borretsch Öl (min. 20% GLA) | Gustav Heess |
| Kakao-Extrakt (20% Polyphenole) | Stefes Ingredients |
| Leinsamen-Extrakt (20% SDG) | |
| Grüntee-Extrakt (75% Catechin + 45% EGCG) | Denk Ingredients |
| Brennnesselwurzel-Extrakt 15:1 | Vivatis Pharma |
| Kürbiskern-Extrakt 20:1 | Frutarom |
| Kürbiskern-Öl | Gustav Heess |
| Sägepalmfrüchte-Öl (10:1, 85-95% fatty acids) | Aceto Health |

Zellen und Chemikalien:

HEK I und HEK II Zellen (Human Embryonic Kidney Cells) wurden freundlicherweise von Prof. R. W. Hartmann, Pharmaceutical & Medicinal Chemistry, Saarland Universität zur Verfügung gestellt. Die HEK I Zellen exprimieren rekombinante 5 alpha Reduktase vom Typ I, während die HEK II Zellen rekombinante 5 alpha Reduktase vom Typ II exprimieren.

Alle verwendeten Chemikalien wurden von kommerziellen Anbieter bezogen und wiesen p. a.-Grad oder die höchste zu erhaltene Reinheit und Qualität auf.

Zellkultur:

HEK I und HEK II Zellen wurden in DMEM (Dulbecco's modified eagle medium, ccpro GmbH) (pH 7.4) mit 10 % FCS (Fetal calf serum, PAN), Penicillin/Streptomycin (100 U/ml und 100 $\mu$g/ml) und 0.5 mg/ml Geneticin-418-sulfat in einer befeuchteten 5 % CO2 Atmosphäre bei 37°C kultiviert.

Herstellung der 5-$\alpha$-Reduktase Typ I und II enthaltenen Zellfraktionen: Für den Zell-freien *in vitro* assays wurden HEK I oder HEK II geerntet, durch Zentrifugation (500 x g) vom Zellkulturmedium befreit und re-suspended in einem Homogenisierungs-Puffer bestehend aus 50 mM Tris-HCl (pH 7.4), 300 mM Saccharose, 0.1 mM EDTA, 10 mM DTT and 100 $\mu$M PMSF. Durch Einfrieren bei - 196 °C und Auftauen auf Eis, wurden die Zellen solubilisiert. Nach einer Inkubations-Zeitraum von 30 min bei 4°C mit 1 mg/ml DNAse in 50 mM MgCl2 und zwischenzeitlich kräftigen Schütteln, wurde das erhaltene Homogenisat bei 20,200 x g in einer gekühlten Zentrifuge für 50 min bei 4°C zentrifugiert. Das Pellet wurde im Homogenisierungs-Puffer re-suspendiert und wieder bei 20,200 x g für 30 Minuten bei at 4°C zentrifugiert. Diese Prozedur wurde zweimal wiederholt. Das Homogenisat-Pellet wurde vom Grund des Röhrchens mit Homogenisierungs-Puffer abgelöst und re-suspendiert durch ultraturax-Mixing bei höchster Geschwindigkeit. Der Protein-Gehalt wurde gemäß der BCA-Methode mit dem kommerziell erhältlichen BCA-Protein Assay Kit von Pierce quantifiziert. Die fraktionierte Zell-Suspension wurde aliquotiert und bei -80°C gelagert.

Herstellung der getesteten Pflanzenextrakte und Öle:

Die Pflanzenextrakte und Öle wurden wie in Tabelle 1 beschrieben bereitgestellt. Stock Lösungen der getesteten Pflanzenextrakte und Öle, sowie der Vergleichssubstanz Finasterid wurden in DMSO hergestellt, außer im Falle von Sägepalmen-Öl, dass in Aceton gelöst wurde (siehe Tabelle 3).
Die Stocklösungen wurden dann nachfolgend mit Hilfe von Assay Puffer in ArbeitsLösungen mit 5-fach höherer Stärke als die beabsichtigte finale Test Konzentration verdünnt. Die Lösemittelkonzentration dieser Arbeitslösungen war 5-fach höher als die beabsichtigte finale Lösemittelkonzentration.
Die verwendeten Test Konzentrationen für die verwendeten Pflanzenextrakte und - öle in Abhängigkeit von deren Löslichkeit sind in Tabelle 3 dargestellt.

Tabelle 3: Stocklösungen der verwendeten Pflanzenextrakte und-öle und der Vergleichssubstanz

| Testsubstanz | Test Konzentrationen [μg/ml] |
|---|---|
| Borretsch Öl (min 20% GLA DAC) | 500, 50, 5 |
| Kakao-Bohnen-Extrakt 20% | 100, 50, 5 |
| Leinsamen-Extrakt 20% | 300, 50, 5 |
| Grüntee-Extrakt 75% Catechin + 45% EGCG | 500, 50, 5 |
| | |
| Brennnesselwurzel-Extrakt 15:1 | 100, 50, 5 |
| Kürbiskern-Extrakt 20:1 | 400, 50, 5 |
| Kürbiskern-Öl | 500, 50, 5 |
| Sägepalmfrüchte-Öl 10:1 | 500, 50, 5 |

Assay Durchführung

HEK I-Inkubationen wurden bei 37 ° C in einer Tris-HCl-EDTA-Puffer-Inkubationsmischung (Assay-Puffer) mit einem Endvolumen von 250 μl durchgeführt, das 0,24 mM NADPH, 250 nM Androstendion, 100 μg Zellhomogenat und Inhibitorverdünnungen enthielt. Bei allen Testsubstanz-Inkubationen betrug die Endkonzentration an organischem Lösungsmittel 1% DMSO (mit Ausnahme von Sägepalmfrüchte-Öl 10: 1: 0,5% Aceton). Finasterid, das als interne Kontrolle verwendet wurde, wurde in DMSO gelöst und in Tris-HCl-EDTA-Puffer verdünnt, um bei Endkonzentrationen von 250 und 1.250 nM getestet zu werden. Mit Lösungsmittel behandelte Kontrollen wurden auf dieselbe Weise behandelt und enthielten 1% DMSO.
HEK II-Inkubationen wurden bei 37 ° C in einer Zitratpuffer-Inkubationsmischung (Assaypuffer) mit einem Endvolumen von 250 μl durchgeführt, das 0,24 mM NADPH, 250 nM Androstendion, 50 μg Zellhomogenat und Inhibitorverdünnungen enthielt. Bei allen Testsubstanz-Inkubationen betrug die Endkonzentration an organischem Lösungsmittel 1% DMSO (mit Ausnahme von Sägepalmfrüchte-Öl 10: 1: 0,5% Aceton). Finasterid, das als interne Kontrolle verwendet wurde, wurde in DMSO gelöst und in Tris-HCl-EDTA-Puffer verdünnt, um bei Endkonzentrationen von 250 und 1.250 nM getestet zu werden. Mit Lösungsmittel behandelte Kontrollen wurden auf dieselbe Weise behandelt und enthielten 1% DMSO.
Die Enzymreaktionen wurden durch Zugabe von zellulären Homogenaten gestartet. Die Inkubationen wurden nach 30 Minuten (HEK I) oder 15 Minuten (HEK II) durch Zugabe eines gleichen Volumens ACN mit dem internen Standard Griseofulvin (ISTD, 0,1 uM) gestoppt. Die Proben wurden bei Raumtemperatur kräftig geschüttelt. Danach wurden die Proben zentrifugiert (5 min bei 4.800 x g, Raum

Flüssig-Chromatographie - Massenspektrometrie Methode

Das HPLC-System bestand aus einer Accela U-HPLC-Pumpe und einem Accela Open-Autosampler (Thermo Fisher Scientific, USA). Die Massenspektrometrie wurde mit einem Q-Exactive-Massenspektrometer (Orbitrap-Technologie mit genauer Masse) durchgeführt, das mit einer beheizten ESI-Schnittstelle (Thermo Fisher Scientific, USA) ausgestattet ist und an einen PC angeschlossen ist, auf dem die Standardsoftware Xcalibur 2.2 ausgeführt wird. Zur Quantifizierung wurde das ISTD-Verfahren (interner Standard) zur Korrektur des Analytenverlusts während der Probenvorbereitung oder des Probeneinlasses angewendet.

Die LC wurde im Gradientenmodus unter Verwendung von wässriger 0,1% Ameisensäure als wässeriger Phase (B) und ACN / 0,1% Ameisensäure als organische Phase (A) durchgeführt; Die Pumpenströmungsrate wurde auf 600 $\mu$l / min eingestellt. Die Trennung wurde auf einer Kinetex-Phenyl-Hexyl, 2,6 $\mu$m, 50 $\times$ 2,1 mm (Phenomenex, Deutschland) Analysensäule mit einer Vorsäule (UHPLC Widepore C4 für Säulen mit 2,1 mm Innendurchmesser) durchgeführt. zur Analyse unter Verwendung der in Tabelle 4 angegebenen Gradienten.

Tabelle 4: HPLC Gradienten

| Mobile Phase | 5α.Reduktase Analyse | | | | | |
|---|---|---|---|---|---|---|
| | 0 min | 0,3 min | 0,7 min | 1,7 min | 1,8 min | 2,5 min |
| A (%) | 5 | 15 | 97 | 97 | 5 | 5 |
| B (%) | 95 | 85 | 3 | 3 | 95 | 95 |

Als MS-Tune-Datei wurde eine generische Tune-Datei verwendet, und als Lock-Masse für die interne Massenkalibrierung wurde das [M+H]$^+$-Ion des Diisooctylphthalats (m/z 391.28429) verwendet, das allgegenwärtig im Lösungsmittelsystem vorhanden ist. Es wurde eine vollständige MS-SIM (m/z: 250-360) -Analyse mit einer Massenauflösung der Orbitrap von 35.000 durchgeführt. Weitere Einstellungen des Analysators waren wie folgt: Max trap injection time 80 ms, sheath gas 40, aux gas 10, sweep gas 2, Kapillarspannung 4 kV, Kapillartemperatur 350°C, H-ESI-Heizertemperatur 350°C.

Tabelle 5 gibt einen Überblick über die MS und Chromatographie-Parameter für die Analyten und den internen Standard (Griseofulvin, ISTD).

Tabelle 5: Überblick über die MS-Bedingungen und Retentionszeiten (positiver Modus H-ESI2)

| Testsubstanz | Molekulargewicht | [M+H]$^+$ (m/z) | Retentionszeit (min) |
|---|---|---|---|
| Androstendion | 286.4 | 287.2008 | 1.3 |
| 5α-Androstendion | 288.4 | 289.2162 | 1.4 |
| ISTD (Griseofulvin) | 352.8 | 353.0786 | 1.3 |

Inhibitionsrate und IC$_{50}$ Wert Kalkulation:

Die Ergebnisse wurden als Peakflächenverhältnis angezeigt (d. h. die Fläche des Analytenpeaks wird durch die Fläche des Peaks des internen Standards geteilt). Die Conversion-Raten wurden nach folgender Formel berechnet:

$$\% \text{ conversion} = \frac{\text{area ratio}_{5\alpha-androstandione}}{\text{area ratio}_{androstenedione} + \text{area ratio}_{5\alpha-androstanedione}} \times 100$$

Die Inhibierungsraten, ausgedrückt als prozentuale Inhibierungswerte relativ zu unbehandelten Kontrollen, wurden berechnet aus den mittleren Umwandlungsraten mit (n = 2) und ohne Inhibitor (n = 2).

Falls anwendbar, wurden die IC$_{50}$-Werte durch lineare Interpolation unter Verwendung der Konzentrationen der Testsubstanzen und des entsprechenden Prozentsatz der Hemmung, die 50% beträgt, berechnet.

$$IC_{50} = (50\% - Low_{Inh}\%)/(High_{Inh}\% - Low_{Inh}\%) \times (High_{Conc} - Low_{Conc}) + Low_{Conc}$$

Ergebnisse:

Acht Testsubstanzen (Pflanzenextrakte und -öle) wurden in vitro auf ihr Hemmpotenzial gegenüber der $5\alpha$-Reduktase-Typen I und II in einem zellfreien Testsystem getestet, wobei Zellhomogenate aus stabil transfizierten HEK293-Zellen isoliert wurden. Der Referenzinhibitor Finasterid diente als positive Kontrolle und wurde parallel getestet.

In einem ersten Schritt wurden alle Testsubstanzen unter Testbedingungen auf ihre Löslichkeit geprüft. Bei den meisten Testsubstanzen war DMSO das geeignete Lösungsmittel, mit Ausnahme von Sägepalmfrüchte-Öl 10: 1, für das Aceton verwendet wurde. Die endgültigen Lösungsmittelkonzentrationen in den Assays waren 1% DMSO bzw. 0,5% Aceton.

Die höchste anwendbare Konzentration für Kürbiskernöl, Grüntee-Extrakt, BorretschÖl und Sägepalmfrüchte-Öl betrug 500 µg/ml. Für Kürbiskernextrakt betrug die höchste anwendbare Testkonzentration 400 µg/ml, während für Leinsamenextrakt die höchste Testkonzentration auf 300 µg / ml begrenzt war. Die höchste anwendbare Löslichkeit von Brennnesselwurzelextrakt, Kakaobohnenextrakt lag bei 100 µg/ml. Für alle Testsubstanzen wurden außerdem weitere Testkonzentrationen 50 µg/ml und 5 µg/ml verwendet.

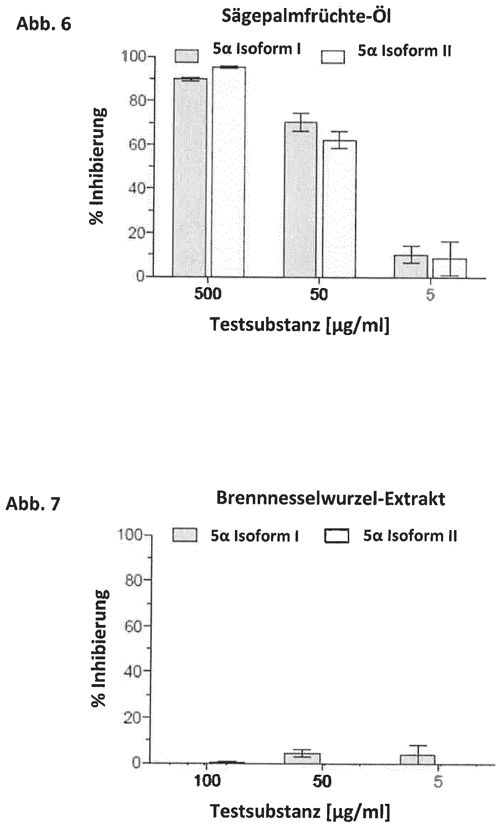

Brennesselwurzelextrakt (Abbildung 7) zeigte kein inhibitorisches Potenzial gegenüber 5a-Reduktase-Isoenzymen bei Konzentrationen bis zu 100 µg / ml.

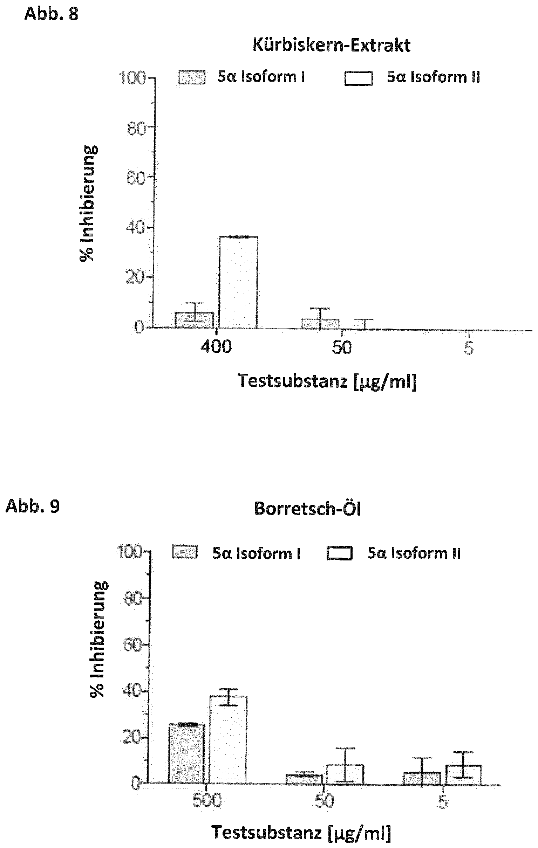

Kürbiskernextrakt zeigte kein inhibitorisches Potential gegenüber 5a-Reduktase Typ I, aber bei der höchsten Testkonzentration von 400 µg / ml wurde die Aktivität des Typs II um 36,6 ± 0,4% gehemmt (Abbildung 8).

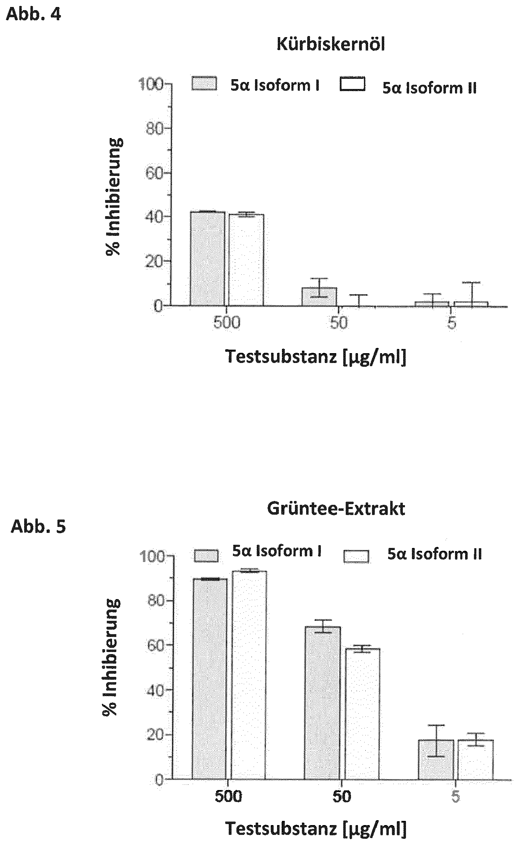

Im Gegensatz dazu hemmte Kürbiskernöl beide Isoformen äquipotent und führte zu 42,7 ± 0,2% Hemmung für Typ I und 41,2 ± 0,8% für Typ II bei 500 µg / ml (Abbildung 4).

Borretschöl war ein moderater Inhibitor beider Isoformen mit einem Maximum der Inhibierung von 25,5 ± 0,7% (Typ I) und 37,6 ± 3,7% (Typ II) bei 500 µg / ml (Abbildung 9).

Kakao-Extrakt war ein sehr potenter Inhibitor beider Isoformen mit einer Inhibierung von 70,6 ± 1,7% (Typ I) und 74,9 ± 0,7% bei 100 µg / ml (Abbildung 2).

Leinsamenextrakt war ein potenter Inhibitor beider Isoformen mit einer Inhibierung von 81,3 ± 1,9% (Typ I) und 92,6 ± 1,0% (Typ II) bei 300 µg / ml (Abbildung 3).

Grüntee-Extrakt war ein sehr potenter Inhibitor beider Isoformen mit einer Inhibierung von 89,7 ± 0,7% (Typ I) und 93,7 ± 0,9% bei 500 µg / ml (Abbildung 5).

Sägepalmfrüchte-Öl 10:1 war ein sehr potenter Inhibitor beider Isoformen mit einer Inhibierung von 89,8 ± 0,7% (Typ I) und 95,4 ± 0,3% bei 500 µg / ml (Abbildung 6).

Im nachfolgenden sind Beispiele der vorliegenden Erfindung angegeben, die zur Veranschaulichung der Erfindung dienen sollen. Die Erfindung soll dabei nicht auf die Bespiele beschränkt sein.

**Beispiele**

**Beispiel1 :**

**Zusammensetzung pro Tag in Form einer Filmtablette und einer Weichgelatinekapsel**

**Filmtablette**

| Pos. | Zutaten | [mg] |
|---|---|---|
| 1. | Kakao-Extrakt | 300 mg |
| 2. | Leinsamen-Extrakt | 300 mg |
| 3. | Mikrokristalline Cellulose | 500 mg |
| 4. | Crosscarmellose Natrium | 11 mg |
| 5. | Magnesiumstearat | 9 mg |
| | **Gewicht Tablettenkern** | **1120 mg** |
| 6. | AquaPolish F dark green | 15 mg |
| | **Gesamtgewicht** | **1135 mg** |

**Weichgelatinekapsel**

| Pos. | Zutaten (Füllung) | [mg |
|------|-------------------|------|
| 1. | Kürbiskernöl | 320 mg |
| 2. | All-rac-alpha-Tocopheryl Acetat | 35 mg |
| 3. | Glyceryl monostearat 40-55 Typ I | 35 mg |
| | **Füllgewicht** | **390 mg** |
| 4. | Gelatine 100/36.5/75 | 252 mg |
| 5. | Glyceryl monostearat | 3 mg |
| | **Gesamtgewicht** | **555 mg** |

**Beispiel 2**

**Zusammensetzung pro Tag in Form einer Lösung (Trinkampulle) und einer Weichgelatinekapsel**

Lösung (Trinkampulle)

| Pos. | Zutaten | [mg] |
|------|---------|------|
| 1. | Kakao-Extrakt | 300 mg |
| 2. | Leinsamen-Extrakt | 300 mg |
| 3. | Fruktose | 3000 mg |
| 4. | Himbeersaft-Konzentrat | 1000mg |
| 5. | Zitronensäure Anhydrat | 90 mg |
| 6. | Kaliumsorbat | 21 mg |
| 7. | Natriumbenzoat | 33 mg |
| 8. | Himbeer Geschmack Symrise | 16 mg |
| 9. | Blanose 7MF-PH | 100 mg |
| 10. | Aqua Purificata | 22390 |
| 11. | NaOH Lölsung 10% | qs |
| 12. | Zitronensäure 10% | qs |
| | | **Gesamt 27250** |

**Weichgelatinekapsel**

| Pos. | Zutaten (Füllung) | [mg |
|------|-------------------|------|
| 1. | Kürbiskernöl | 320 mg |
| 2. | All-rac-alpha-Tocopheryl Acetat | 35 mg |
| 3. | Glyceryl monostearat 40-55 Typ I | 35 mg |
| | **Füllgewicht** | **390 mg** |
| 4. | Gelatine 100/36.5/75 | 252 mg |
| 5. | Glyceryl monostearat | 3 mg |

(fortgesetzt)

| Pos. | Zutaten (Füllung) | [mg |
|------|-------------------|-----|
| | **Gesamtgewicht** | **555 mg** |

Beispiel 3:

**Zusammensetzung pro Tag in Form zweier Filmtabletten und einer Weichgelatinekapsel**

Filmtablette (2 pro Tag)

| Pos. | Ingredients | [mg]/ Tablette |
|------|-------------|----------------|
| 1. | Kakao-Extrakt | 150 mg |
| 2. | Leinsamen-Extrakt | 150 mg |
| 3. | Grüntee-Extrakt | 150 mg |
| 4. | Curcumin | 160 mg |
| 5. | Zinkoxid | 4,45 mg |
| 6. | Vitamin D 3 100.000I.U. | 4,8 mg |
| 7. | Mikrokristalline Cellulose | 360,75 mg |
| 8. | Crosscarmellose Natrium | 11 mg |
| 9. | Magnesiumstearat | 9 mg |
| | **Gewicht Tablettenkern** | **1000 mg** |
| 10. | AquaPolish F dark green | 10 mg |
| | **Gesamtgewicht** | **1010 mg** |

Weichgelatinekapsel

| Pos. | Zutaten (Füllung) | [mg] |
|------|-------------------|------|
| 1. | Kürbiskernöl | 320 mg |
| 2. | Sägepalmfrüchte-Öl | 320 mg |
| 3. | Lycopin synth. | 3 mg |
| 4. | All-rac-alpha-Tocopheryl Acetat | 35 mg |
| 5. | Glyceryl monostearate 40-55 Typ I | 35 mg |
| | **Füllgewicht** | **713 mg** |
| 6. | Gelatine 100/36.5/75 | 460mg |
| 7. | Glyceryl monostearat | 5 mg |
| | **Gesamtgewicht** | **606 mg** |

Beispiel 4:

**Zusammensetzung pro Tag in Form eines Pulvers (Sachet) und einer Weichgelatinekapsel**

Sachet Pulver

| Pos. | Zutaten | [mg] |
|---|---|---|
| 1. | Kakao-Extrakt | 300 mg |
| 2. | Leinsamen-Extrakt 33% SDG | 300 mg |
| 3. | Grüntee-Extrakt | 300 mg |
| 4. | Curcumin | 320 mg |
| 5. | Zinkoxid | 8,9 mg |
| 6. | Vitamin D 3 100.000I.U. | 9,6 mg |
| 7. | Maltodextrin | 3978,5 mg |
| 8. | Äpfelsäure, fein | 400 mg |
| 9. | Zitronensäure Anhydrat | 200 mg |
| 10. | Sucralose | 12 mg |
| 11. | Acesulfam | 11 mg |
| 12. | MCT Powder | 60 mg |
| 13. | Sipemat 50 S | 50 mg |
| 14. | Magnesiumhydroxidcarbonat, leicht | 50 mg |
| 15. | Orangenaroma Symrise | |
| **Gesamtgewicht 6000 mg** | | |

Weichgelatinekapsel

| Pos. | Zutaten (Füllung) | [mg] |
|---|---|---|
| 1. | Kürbiskernöl | 320 mg |
| 2. | Sägepalmfrüchte-Öl | 320 mg |
| 3. | Lycopin synth. | 3 mg |
| 4. | All-rac-alpha-Tocopheryl Acetat | 35 mg |
| 5. | Glyceryl monostearate 40-55 Typ I | 35 mg |
| | **Füllgewicht** | **713 mg** |
| 6. | Gelatine 100/36.5/75 | 460mg |
| 7. | Glyceryl monostearat | 5 mg |
| | **Gesamtgewicht** | **606 mg** |

**Patentansprüche**

1. Oral einzunehmende Zusammensetzung, insbesondere geeignet als Nahrungsergänzungsmittel oder diätetisches Lebensmittel, wobei die Zusammensetzung bezogen auf eine Tagesdosis der Zusammensetzung eine Kombination aus 100 -500 mg Kakao-Extrakt, 100 - 500 mg Leinsamen-Extrakt und 100 - 1000 mg Kürbiskernöl umfasst.

2. Oral einzunehmende Zusammensetzung nach Anspruch 1, wobei die Zusammensetzung bezogen auf eine Tagesdosis der Zusammensetzung zusätzlich 2- 100 $\mu$g Vitamin D und/oder 2-20 mg Zink umfasst.

3. Oral einzunehmende Zusammensetzung nach Anspruch 1 oder 2, wobei die Zusammensetzung bezogen auf eine Tagesdosis der Zusammensetzung zusätzlich 100 - 500 mg Grüntee-Extrakt und/oder 100 -500 mg Sägepalmfrüchte-Öl umfasst.

4. Oral einzunehmende Zusammensetzung nach Anspruch 1 bis 3, wobei die Zusammensetzung bezogen auf eine Tagesdosis der Zusammensetzung zusätzlich 100 - 400 mg Curcumin/Curcuma-Extrakt und/oder 0,5- 6 mg Lycopin umfasst.

5. Oral einzunehmende Zusammensetzung nach Anspruch 1-4, wobei die Zusammensetzung in Form einer, zweier oder mehrerer gleicher oder unterschiedlicher Darreichungsformen vorliegt und wobei die jeweilige Darreichungsform in Form einer Kapsel, Lösung, Tablette, Pulver, Emulsion, Granulat, Riegel, Toffee oder in anderer kaubarer, schluckbarer oder trinkbarer Form vorliegt.

6. Oral einzunehmende Zusammensetzung nach Anspruch 5, wobei die Zusammensetzung in Form zweier Darreichungsformen vorliegt wobei die eine Darreichungsform in Form einer einzigen Kapsel (beispielsweise Weichgelatinekapsel) vorliegt und die andere Darreichungsform in Form einer oder mehrerer Lösung(en), einer oder mehrerer Tablette(n), einer oder mehrerer Pulver, einer oder mehrerer Emulsion(en), einem oder mehrerer Granulat(e), einen oder mehrerer Riegel, einen oder mehrerer Toffee oder in einer oder mehrerer anderer kaubarer, schluckbarer oder trinkbarer Form(en) vorliegt und wobei die Kapsel eine Tagesdosis von 100 - 1000 mg Kürbiskernöl, bevorzugt 200 - 700 mg Kürbiskernöl, besonders bevorzugt 250 - 400 mg Kürbiskernöl enthält und die andere Darreichungsform eine Tagesdosis von 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200 - 400 mg Leinsamenextrakt, besonders bevorzugt 250 - 350 mg Leinsamenextrakt enthält.

7. Oral einzunehmende Zusammensetzung nach Anspruch 5 bis 6, wobei die Zusammensetzung in Form einer einzigen Kapsel (beispielsweise Weichgelatinekapsel) und einer einzigen Tablette vorliegt, wobei die Kapsel eine Tagesdosis von 100 - 1000 mg Kürbiskernöl, bevorzugt 200- 700 mg Kürbiskernöl, besonders bevorzugt 250 - 400 mg Kürbiskernöl enthält und die Tablette eine Tagesdosis von 100 - 500 mg Kakao-Extrakt, bevorzugt 200 - 400 mg Kakao-Extrakt, besonders bevorzugt 250 - 350 mg Kakao-Extrakt sowie 100 - 500 mg Leinsamenextrakt, bevorzugt 200-400 mg Leinsamenextrakt, besonders bevorzugt 250 - 350 mg Leinsamenextrakt enthält.

8. Nahrungsergänzungsmittel oder Diätetisches Lebensmittel in Form einer Zusammensetzung gemäß Anspruch 5-7.

9. Nahrungsergänzungsmittel oder Diätetisches Lebensmittel nach Anspruch 8, wobei die Zusammensetzung innerhalb eines Gebindes, eines Kits oder einer Produkt- oder Verpackungseinheit vorliegt.

10. Nicht-therapeutische Verwendung einer oral einzunehmenden Zusammensetzung nach Anspruch 1-4 zur Verminderung vom Haarausfall und zur Verbesserung des Haarwuchses bei Androgenetischer Alopezie.

11. Nicht-therapeutische Verwendung einer oral einzunehmenden Zusammensetzung nach Anspruch 1-4 zur diätetischen Unterstützung einer Behandlung von Haarausfall und zur diätetischen Unterstützung zur Verbesserung des Haarwuchses bei Androgenetischer Alopezie.

12. Nicht-therapeutische Verwendung einer oral einzunehmenden Zusammensetzung nach Anspruch 1- 4 als Nahrungsergänzungsmittel zur Verminderung vom Haarausfall und zur Verbesserung des Haarwuchses bei Androgenetischer Alopezie.

13. Nicht-therapeutische Verwendung einer Kombination aus 100 - 500 mg Kakao-Extrakt, bevorzugt 200 - 400 mg Kakao-Extrakt, besonders bevorzugt 250 - 350 mg Kakao-Extrakt und 100 - 500 mg Leinsamen-Extrakt, bevorzugt 200 - 400 mg Leinsamenextrakt, besonders bevorzugt 250 - 350 mg Leinsamenextrakt und 100 - 1000 mg Kürbiskernöl, bevorzugt 200 - 700 mg Kürbiskernöl, besonders bevorzugt 250 - 400 mg Kürbiskernöl zur oralen Verabreichung pro Tag oder als Tagesdosis zur diätetischen Unterstützung einer Behandlung von Haarausfall und zur diätetischen Unterstützung zur Verbesserung des Haarwuchses bei Androgenetischer Alopezie oder zur Verminderung von Haarausfall und zur Verbesserung des Haarwuchses bei Androgenetischer Alopezie.

14. Nicht-therapeutische Verwendung nach Anspruch 11 wobei das Kürbiskernöl in Form einer Kapsel, vorzugsweise einer Weichgelantinekapsel vorliegt und der Kakao-Extrakt und der Leinsamenextrakt in Form einer oder mehrerer Lösung(en), einer oder mehrerer Tablette(n), einer oder mehrerer Pulver, einer oder mehrerer Emulsion(en), einem oder mehrerer Granulat(e), einer oder mehrerer Riegel, einer oder mehrerer Toffee oder in einer oder mehrerer anderer kaubarer, schluckbarer oder trinkbarer Form(en) vorliegt.

15. Pharmazeutische Zusammensetzung nach Anspruch 1-4 zur Verwendung bei der Behandlung der androgenetischen

Alopezie.

**Abb. 1**

**Abb. 2**

### Kakao - Extrakt

**Abb. 3**

### Leinsamen-Extrakt

**Abb. 4**

Kürbiskernöl

**Abb. 5**

Grüntee-Extrakt

Abb. 6

## Sägepalmfrüchte-Öl

Abb. 7

## Brennnesselwurzel-Extrakt

**Abb. 8**

**Kürbiskern-Extrakt**

**Abb. 9**

**Borretsch-Öl**

Europäisches Patentamt

European Patent Office

Office européen des brevets

## EUROPÄISCHER TEILRECHERCHENBERICHT

nach Regel 62a und/oder 63 des Europäischen Patentübereinkommens. Dieser Bericht gilt für das weitere
Verfahren als europäischer Recherchenbericht.

Nummer der Anmeldung

EP 19 18 5940

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| X | Gnpd: "Beauty Skin Food Supplement Product Description", , 1. Mai 2019 (2019-05-01), Seiten 1-4, XP055637161, Gefunden im Internet: URL:www.gnpd.com [gefunden am 2019-10-29] * das ganze Dokument * ----- | 1-7, 10-15 | INV. A23L33/105 A23L33/115 A61K45/00 A61P17/14 A61Q7/00 A61K36/42 A61K36/55 |
| X | DATABASE GNPD [Online] MINTEL; 14. April 2015 (2015-04-14), anonymous: "Chocolate Cacao High Protein for Weight Loss", XP055637169, gefunden im www.gnpd.com Database accession no. 3088363 * das ganze Dokument * ----- -/-- | 1-5 | |

**RECHERCHIERTE SACHGEBIETE (IPC)**

A23L
A61Q
A61P
A61K

### UNVOLLSTÄNDIGE RECHERCHE

Die Recherchenabteilung ist der Auffassung, daß ein oder mehrere Ansprüche, den Vorschriften des EPÜ nicht entspricht bzw. entsprechen, so daß nur eine Teilrecherche (R.62a, 63) durchgeführt wurde.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| München | 30. Oktober 2019 | Barac, Dominika |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.............................................................
& : Mitglied der gleichen Patentfamilie, übereinstimmendes Dokument

EPO FORM 1503 03.82 (P04E09)

Seite 1 von 5

Europäisches
Patentamt

European
Patent Office

Office européen
des brevets

**EUROPÄISCHER
TEILRECHERCHENBERICHT**

Nummer der Anmeldung

EP 19 18 5940

| | | **EINSCHLÄGIGE DOKUMENTE** | | **KLASSIFIKATION DER ANMELDUNG (IPC)** |
|---|---|---|---|---|
| Kategorie | | Kennzeichnung des Dokuments mit Angabe, soweit erforderlich der maßgeblichen Teile | Betrifft Anspruch | |
| X | | DATABASE GNPD [Online] MINTEL; 3. September 2018 (2018-09-03), anonymous: "Wholefood Nutrition Shake with Cocoa", XP055637174, gefunden im www.gnpd.com Database accession no. 5948985 * das ganze Dokument * | 1,2,5 | |
| X | | DATABASE GNPD [Online] MINTEL; 1. Februar 2016 (2016-02-01), anonymous: "Oat Berry with Dark Chocolate Bar For Maternal Nutrition And Lactation", XP055637172, gefunden im www.gnpd.com Database accession no. 3762813 * das ganze Dokument * | 1,2,5 | **RECHERCHIERTE SACHGEBIETE (IPC)** |
| Y | | DATABASE GNPD [Online] MINTEL; 1. Juli 2006 (2006-07-01), anonymous: "Nutrional Supplement to Reduce Hair Loss", XP055636790, gefunden im www.gnpd.com Database accession no. 650793 * das ganze Dokument * | 1-7, 10-15 | |
| Y | | DATABASE GNPD [Online] MINTEL; 29. April 2003 (2003-04-29), anonymous: "System 1 Hairgrow Capsules", XP055636800, gefunden im www.gnpd.com Database accession no. 203529 * das ganze Dokument * | 1-7, 10-15 | |

-/--

EPO FORM 1503 03.82 (P04C12)

Europäisches
Patentamt

European
Patent Office

Office européen
des brevets

**EUROPÄISCHER
TEILRECHERCHENBERICHT**

Nummer der Anmeldung

EP 19 18 5940

| | | **EINSCHLÄGIGE DOKUMENTE** | | KLASSIFIKATION DER ANMELDUNG (IPC) |
|---|---|---|---|---|
| Kategorie | Kennzeichnung des Dokuments mit Angabe, soweit erforderlich der maßgeblichen Teile | | Betrifft Anspruch | |
| Y | DATABASE GNPD [Online] MINTEL; 20. April 2015 (2015-04-20), anonymous: "Anti Hair Loss Supplement", XP055636552, gefunden im www.gnpd.com Database accession no. 3105925 * Zusammenfassung * ----- | | 1-7, 10-15 | |
| Y | Anonymous: "Ell-Cranell Haarfülle+ für Männer Tabletten + Kapseln, 180 Stück (90x Tablette + 90x Kapsel) ¦ heise online Preisvergleich / Deutschland", , 19. Mai 2017 (2017-05-19), Seiten 1-2, XP055636984, Gefunden im Internet: URL:https://www.heise.de/preisvergleich/ell-cranell-haarfuelle-fuer-maenner-tabletten-kapseln-90x-tablette-90x-kapsel-a1626648.html [gefunden am 2019-10-29] * das ganze Dokument * ----- | | 1-7, 10-15 | |
| | | | | **RECHERCHIERTE SACHGEBIETE (IPC)** |
| T | Ananoymous: "Ell-Cranell re-balance Haarfülle+ für Männer", , 29. Oktober 2019 (2019-10-29), Seiten 1-1, XP055636989, Gefunden im Internet: URL:www.heise.de [gefunden am 2019-10-29] * das ganze Dokument * ----- | | | |
| Y | US 2008/064765 A1 (BIRNBAUM JACOB [US]) 13. März 2008 (2008-03-13) * Beispiele 1-5 * * Ansprüche 1-12 * ----- -/-- | | 1-7, 10-15 | |

EPO FORM 1503 03.82 (P04C12)

Europäisches
Patentamt

European
Patent Office

Office européen
des brevets

**EUROPÄISCHER
TEILRECHERCHENBERICHT**

Nummer der Anmeldung

EP 19 18 5940

| | EINSCHLÄGIGE DOKUMENTE | | KLASSIFIKATION DER ANMELDUNG (IPC) |
|---|---|---|---|
| Kategorie | Kennzeichnung des Dokuments mit Angabe, soweit erforderlich der maßgeblichen Teile | Betrifft Anspruch | |
| Y | Anonymous: "10 types of nutrients for your hair to THRIVE ON", , 10. September 2017 (2017-09-10), Seiten 1-16, XP055637287, England Gefunden im Internet: URL:https://web.archive.org/web/2017091010 0028/http://www.signatureshelley.com/new-blog/10nutrients [gefunden am 2019-10-30] * das ganze Dokument * ----- | 1-7, 10-15 | |
| Y | Arielle Tschinkel: "Foods to eat for healthy hair -Insiderbest-foods-for-healthy-hair-2018-4 #", , 7. Mai 2018 (2018-05-07), Seiten 1-18, XP055637213, Gefunden im Internet: URL:www.insider.com [gefunden am 2019-10-29] * das ganze Dokument * ----- | 1-7, 10-15 | **RECHERCHIERTE SACHGEBIETE** (IPC) |
| Y | PARVIN RAMAK ET AL: "The beneficial effects of Pumpkin ( Cucurbita pepo L.) seed oil for health condition of men", FOOD REVIEWS INTERNATIONAL, Bd. 35, Nr. 2, 28. Juni 2018 (2018-06-28), Seiten 166-176, XP055636407, Philadelphia, USA ISSN: 8755-9129, DOI: 10.1080/87559129.2018.1482496 * das ganze Dokument * ----- | 1-7, 10-15 | |

-/--

EPO FORM 1503 03.82 (P04C12)

**Europäisches Patentamt**
**European Patent Office**
**Office européen des brevets**

**EUROPÄISCHER
TEILRECHERCHENBERICHT**

Nummer der Anmeldung

EP 19 18 5940

| | EINSCHLÄGIGE DOKUMENTE | | KLASSIFIKATION DER ANMELDUNG (IPC) |
|---|---|---|---|
| Kategorie | Kennzeichnung des Dokuments mit Angabe, soweit erforderlich der maßgeblichen Teile | Betrifft Anspruch | |
| Y | Anonymous: "Chocolate for Hair Loss ¦ Theradome", , 12. Juli 2018 (2018-07-12), Seiten 1-9, XP055636474, Gefunden im Internet: URL:https://www.theradome.com/chocolate-hair-loss/ [gefunden am 2019-10-28] * das ganze Dokument * ----- | 1-7, 10-15 | |
| Y | Doctor.Ndtv.Com: "Tips for Long, Thick Hair: These Nutrients Are What You Need", , 7. Mai 2018 (2018-05-07), Seiten 1-6, XP055636500, Gefunden im Internet: URL:https://doctor.ndtv.com/nutrition/want-long-thick-and-luscious-hair-these-nutrients-are-what-you-need-1820222 [gefunden am 2019-10-28] * das ganze Dokument * ----- | 1-7, 10-15 | **RECHERCHIERTE SACHGEBIETE** (IPC) |
| A | Interdisziplinäres Management Der Androgenetischen Alopezie: "Stellungnahme der GD Gesellschaft für Dermopharmazie e. V", , 22. März 2010 (2010-03-22), Seiten 1-12, XP055637010, Gefunden im Internet: URL:http://www.gd-online.de/german/veranstalt/images2010/GD_Stellungnahme_Management_Alopezie_2010.pdf [gefunden am 2019-10-29] * das ganze Dokument * ----- | 1-7, 10-15 | |

EPO FORM 1503 03.82 (P04C12)

Seite 5 von 5

Europäisches Patentamt

European Patent Office

Office européen des brevets

**UNVOLLSTÄNDIGE RECHERCHE
ERGÄNZUNGSBLATT C**

**Nummer der Anmeldung**

EP 19 18 5940

Vollständig recherchierbare Ansprüche:
    1-7, 10-15

Nicht recherchierte Ansprüche:
    8, 9

Grund für die Beschränkung der Recherche:

Gemäß Regel 62(a)(1) EPÜ wurde die Anmelderin am 19.09.19 aufgefordert den zu recherchierbaren Gegenstand anzugeben.
In seinem Antwortschreiben vom 17.10.19 hat der Anmelder angegeben, dass der die recherche basierend auf dem unabhängigen Produktanspruch 1 durchgeführt werden soll.
Folglich basiert die durchgeführte Suche wie von der Anmelderin angegeben, auf dem Gegenstand des Anspruchs 1, sprich es wurde die Ansprüche 1-7, 10-15 recherchiert.
Der Gegenstand des unabhängigen Anspruchs 8, sowei des abhängigen Anspruchs 9 wurde von der Recherche ausgeschlossen.

**ANHANG ZUM EUROPÄISCHEN RECHERCHENBERICHT
ÜBER DIE EUROPÄISCHE PATENTANMELDUNG NR.**

EP 19 18 5940

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-10-2019

| Im Recherchenbericht angeführtes Patentdokument | Datum der Veröffentlichung | Mitglied(er) der Patentfamilie | Datum der Veröffentlichung |
|---|---|---|---|
| US 2008064765 A1 | 13-03-2008 | KEINE | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004039983
- WO 2007034323 A
- US 20060009430 A
- WO 00256269 A


**In der Beschreibung aufgeführte Nicht-Patentliteratur**

- **BLUMEYER et al.** *J Dtsch Dermatol Ges,* 2011, vol. 9 (6), 1-57
- **MOWSZOWICZ I et al.** *J Clin Endocrinol Metab,* 01. Juni 1983, vol. 56 (6), 1209-1213
- **GODOY A et al.** *Prostate,* 07. Juli 2011, vol. 1 (10), 1033-1046
- **YAMANA K et al.** *Horm Mol Biol Clin Investig.,* 01. August 2010, vol. 2 (3), 293-9
- **FERTIG et al.** *Dermatol Online J,* 2017, vol. 23, 11
- **AZZOUNI et al.** *Advances in Urology, 2012,* 2012, 530121
- **LIU et al.** *J Sex Med,* 2016, vol. 13 (9), 1297-310
- **KIGURADZE et al.** *PeerJ,* 2017
- **ROSSI et al.** einer vergleichenden Studie mit Finasterid zeigen, dass ein Extrakt aus Sägepalmfrüchten (Serenoa repens) eine Verbesserung der androgenetischen Alopezie bewirken kann. *Int J Immunopathol Pharmacol.,* Oktober 2012, vol. 25 (4), 1167-73
- **PRAGER N et al.** *J Altern Complement Med.,* April 2002, vol. 8 (2), 143-52
- **KWON et al.** *Phytomedicine,* August 2007, vol. 14 (7-8), 551-555
- **CHO et al.** *Evidence-Based Complementary and Alternative Medicine,* vol. 2014